# EUROPEAN PATENT SPECIFICATION

(11) **EP 3 972 564 B1**
(45) Date of publication and mention of the grant of the patent: **02.04.2025**
(21) Application number: 20734804.6
(22) Date of filing: 25.05.2020
(51) Int. Cl.: A61K 9/00, A61K 9/06, A61K 9/08, A61K 47/10, A61K 47/36, A61P 27/02

(54) **OPHTHALMIC COMPOSITION DEVOID OF CHELATORS FOR USE IN A METHOD FOR THE TREATMENT OF AN OCULAR DISORDER**
OPHTHALMISCHE ZUSAMMENSETZUNG OHNE CHELATOREN ZUR VERWENDUNG IN EINEM VERFAHREN ZUR BEHANDLUNG EINER AUGENERKRANKUNG
COMPOSITION OPHTALMIQUE DÉPOURVUE DE CHÉLATEURS DESTINÉE À ÊTRE UTILISÉE DANS UN PROCÉDÉ DE TRAITEMENT D'UN TROUBLE OCULAIRE

(30) Priority: 23.05.2019 IT 201900007186
(43) Date of publication of application: 30.03.2022
(73) Proprietor: NTC S.r.l., 20124 Milano (IT)
(72) Inventor: MARCELLONI, Luciano, 20124 Milano (MI) (IT); BERTOCCHI, Federico, 20124 Milano (MI) (IT)
(74) Representative: Benedetto, Marco
(86) International application number: PCT/IB2020/054940
(87) International publication number: WO 2020/234854

(56) References cited:
- JP-A- 2013 144 671
- KR-A- 20160 096 376
- US-A1- 2010 234 318
- WEI G ET AL: "Thermosetting gels with modulated gelation temperature for ophthalmic use: the rheological and gamma scintigraphic studies", JOURNAL OF CONTROLLED RELEASE, ELSEVIER, AMSTERDAM, NL, vol. 83, no. 1, 18 September 2002 (2002-09-18), pages 65 - 74, XP004380060, ISSN: 0168-3659, DOI: 10.1016/S0168-3659(02)00175-X

## Description

The present invention regards an ophthalmic composition comprising a mixture for use in a method for the treatment of a disorder or a disease associated with a corneal epithelial defect and/or a conjunctival defect in a subject.

A pharmaceutical composition for ophthalmic use is known from the prior document EP1948131A2. Said composition is devoid of phosphate and it comprises a calcium chelating agent and a viscosity adjusting compound.

According to document EP1948131A2, such composition has: 1) a low concentration (<7 mmol/l) of phosphate ions which reduces the possibility that such ions can bind to calcium ions, present on the cornea, with the formation of poorly soluble and abrasive calcium phosphate deposits; 2) a chelating agent for removing the calcium ions physiologically present at the cornea; 3) a viscosity adjusting compound which allows to exert a mechanical lubricating action between the cornea and a respective eyelid.

Nevertheless, the prior art known from document EP1948131A2 reveals some limits and drawbacks.

In particular, the inventors of the present invention observed that the pharmaceutical composition obtained according to the disclosure of document EP1948131A2 may have an astringent effect on the eye. Without wishing to provide a scientific explanation for the phenomenon, the present inventors observed that the presence of a chelating agent, such as for example EDTA, does not sequester calcium ions alone, but also a variety of other ions present on the cornea (such as for example the selenium ion, and/or the zinc ion, and/or the selenium ion), in a non-specific manner. Therefore, the chelating agent, such as for example EDTA, with its sequestering action, generates large hypotonic areas which last for the entire period of time of residence of the chelating agent on the cornea. A subtraction (sequestration) of such ions, especially of the bivalent ions (such as for example calcium, but also the zinc ion and the selenium ion) alters the tonicity of the eye, which can manifest itself as an eye burning sensation, a very uncomfortable sensation for the users of a such ophthalmic composition.

The scientific article WEI G. ET AL: "Thermosetting gels with modulated gelation temperature for ophthalmic use: the rheological and gamma scintigraphic studies" (JOURNAL OF CONTROLLED RELEASE, ELSEVIER, AMSTERDAM, NL, vol. 83, no. 1) is a study aimed at investigating the gelation temperature, rheological properties and mucoadhesion of compositions comprising one or two viscosity adjusting agents (Pluronic^{®} F127 and/or Pluronic^{®} F68) in combination with sodium hyaluronate (HA-Na). All the tests carried out in this study provide for an initial dissolution of HA-Na in an isotonic saline solution in phosphate buffer solution (PBS). Phosphate buffer is always present. Such study drew the conclusion that the presence of HA-Na in the tested gels does not produce improvements in relation to ocular retention, since sodium hyaluronate is considered to be a cause of weakening of gel hardness.

Furthermore, the presence of a single Pluronic^{®} F127 agent in high concentrations (for example of 25%) is considered poorly suitable in the ophthalmic field since it is capable of forming a solid gel at 17,5°C instead of forming gel in the ocular site of application at physiological temperature.

Document US 2010/234318 A1 illustrates an ophthalmic composition containing alginic acid, or a salt thereof, and hyaluronic acid, or a salt thereof. The composition has an increased capacity to remain on the mucosa of the eye. Such composition is used to relieve or improve symptoms, such as corneal dryness or inflammation, which often occur in users of contact lenses. The compositions exemplified in Example 8 and Example 13 of such document (Table 10) have an excessively acidic pH value - respectively having pH = 5 and pH = 5.5 - to be used in the ophthalmic field. Furthermore, the compositions exemplified in Example 8, Example 13 and Example 33 of such document are devoid of tris(hydroxymethyl)aminomethane (trometamol, or tromethamine CAS 77-86-1)).

The need is therefore felt to have an ophthalmic composition which is more tolerable with respect to the known compositions, considering the same effectiveness.

After a long and intense research and development activity, the Applicant developed an ophthalmic composition, preferably an ophthalmic solution, capable of providing an adequate response to the limits, drawbacks and problems currently existing in the prior art.

Forming an object of the present invention is an ophthalmic composition comprising a mixture for use in a method for the treatment of a disorder or a disease associated with a corneal epithelial defect and/or a conjunctival defect in a subject, having the characteristics as defined in the attached claims.

As said above, the composition subject of the present invention is devoid of chelating agents such as for example EDTA or salts thereof such as EDTA sodium salt. By way of example, the chelating agents conventionally used in the ophthalmologic field are citrate salts, citric acid, diethylene diamine tetraacetic acid (EDTA; CAS N° 60-00-4) or salts thereof, triethylene glycol diamine tetraacetic acid (EGTA, CAS N° 67-42-5), or combinations thereof and salts thereof. All these chelating agents are not present in the composition and/or mixture of the present invention.

On the other hand, the inventors of the present invention found that the absence of EDTA, in the composition subject of the present invention, produces unfavourable conditions for the gelation (which allows the liquid composition to become a gel) of the viscosity adjusting agents, especially due to the greater presence of ions that hinder the formation of a gel, at physiological temperature. To overcome this drawback, the inventors of the present invention selected, from among the many compounds tested, the tris(hydroxymethyl)aminomethane (trometamol, or tromethamine CAS 77-86-1) compound. Tris(hydroxymethyl)aminomethane does not release ions in aqueous solution and, together with boric acid or salts thereof, is capable of forming a buffer system that allows to adjust the pH to a physiological value by hindering interference in the viscosity of the composition and the formation of a gel on the surface of the eyeball.

The ophthalmic composition, subject of the present invention is devoid of chelating agents (such as for example EDTA or salts thereof such as sodium salt) and it comprises a mixture. The mixture comprises, or alternatively, consists of:
(a) at least two viscosity adjusting agents; a first viscosity adjusting agent (a.1) selected from among the group comprising or, alternatively, consisting of hyaluronic acid, a salt thereof and mixtures thereof; a second viscosity adjusting agent (a.2) comprising or, alternatively, consisting of an ethylene oxide/propylene oxide block copolymer,
(b) a buffer agent which is a mixture comprising, or alternatively, consisting of boric acid and tris(hydroxymethyl)aminomethane for adjusting the pH of the ophthalmic composition to a value comprised from 6,5 to 8 ±0,1, preferably comprised from 6,8 to 7,2 ±0,1 or ± 0,2, for example, a pH value of 7 ± 0,1, ± 0,2,
(c) preferably one or more pharmaceutical grade additives and excipients, and
(d) water.

According to an embodiment, the composition is devoid of phosphates, i.e. devoid of phosphate ions, monohydrogen phosphate ions and/or dihydrogen phosphate ions.

As regards the terminology used in this description, it is specified that the expression "devoid of phosphates" is used to indicate the total absence of phosphate ions, monohydrogen phosphate ions and/or dihydrogen phosphate ions in the composition and/or mixture, or a presence of phosphates at an amount comprised from greater than zero to 7 mmol/litre, preferably from greater than zero to 3 mmol/litre, even more preferably from greater than zero to 1 mmol/litre of phosphates, i.e. devoid of phosphate ions, of monohydrogen phosphate ions and/or dihydrogen phosphate ions.

The ophthalmic composition, subject of the present invention is devoid of chelating agents (such as for example EDTA or salts thereof such as sodium salt) of phosphates and it comprises a mixture. The mixture comprises, or alternatively, consists of:
(a) at least two viscosity adjusting agents; a first viscosity adjusting agent (a.1) selected from among the group comprising or, alternatively, consisting of hyaluronic acid, a salt thereof and mixtures thereof; a second viscosity adjusting agent (a.2) comprising or, alternatively, consisting of an ethylene oxide/propylene oxide block copolymer,
(b) a buffer agent which is a mixture comprising, or alternatively, consisting of boric acid and tris(hydroxymethyl)aminomethane for adjusting the pH of the ophthalmic composition to a value comprised from 6,5 to 8 ±0,1, preferably comprised from 6,8 to 7,2 ±0,1 or ± 0,2, for example, a pH value of 7 ± 0,1, ± 0,2,
(c) preferably one or more pharmaceutical grade additives and excipients, and
(d) water, said composition and said mixture being devoid of chelating agents and of phosphates.

The buffer agent (b), used in combination with (a) in the mixture of the present invention, is a mixture comprising or consisting of boric acid and tris(hydroxymethyl)aminomethane (trometamol or tromethamine; CAS N° 77-86-1) and it is present, in the composition, at an amount by weight comprised from 0,01% to 1%, preferably comprised from 0,035% to 0,8%, more preferably comprised from 0,05% to 0,7%, even more preferably comprised from 0,08% to 0,5%, for example comprised from 0,1% to 0,32%, with respect to the total weight of the composition.

In the composition, boric acid is present at an amount by weight comprised from 0,01% to 0,05%, preferably from 0,02% to 0,04%, even more preferably from 0,03% to 0,035%, with respect to the total weight of said composition.

In the composition, tris(hydroxymethyl)aminomethane (trometamol or tromethamine; CAS N° 77-86-1) is present at an amount by weight comprised from 0,01% to 0,12%, preferably from 0,04% to 0,1%, even more preferably from 0,06% to 0,08%, with respect to the total weight of said composition.

In the composition, boric acid and tris(hydroxymethyl)aminomethane (trometamol or tromethamine; CAS N° 77-86-1) are present at a ratio by weight comprised from 1:1 to 1:4, preferably from 1:2 to 1:3.

The composition subject of the present invention is preferably in the form of liquid eye drops, or gel, cream or ointment or foam eye drops.

According to an embodiment, the composition subject of the present invention has an osmolality, at a temperature of 25°C, comprised from 200 mOsm/Kg to 950 mOsm/Kg, preferably comprised from 220 mOsm/Kg to 880 mOsm/kg, even more preferably comprised from 250 mOsm/Kg to 800 mOsm/Kg; for example an osmolality comprised from 300 mOsm/Kg to 600 mOsm/Kg.

Thus, according to different embodiments, said composition can be a hypotonic composition (for example with an osmolality value of about from 225 mOsm/kg to 275 mOsm/kg, or comprised from 220 mOsm/kg to 265 mOsm/kg, preferably comprised from 230 mOsm/kg to 260 mOsm/kg, even more preferably comprised from 235 mOsm/Kg to 255 mOsm/Kg, for example 250 mOsm/Kg), a hypertonic composition (for example with an osmolality value greater than from 350 mOsm/Kg to 850 mOsm/Kg or comprised from 700 mOsm/Kg to 1000 mOsm/Kg, preferably comprised from 750 mOsm/Kg to 930 mOsm/Kg, even more preferably comprised from 800 mOsm/Kg to 910 mOsm/Kg, for example 880 mOsm/Kg) or an isotonic composition with the tear liquid (for example with an osmolality value of about from 275 mOsm/Kg to 325 mOsm/Kg or comprised from 270 mOsm/Kg to 335 mOsm/Kg, preferably comprised from 280 mOsm/Kg to 330 mOsm/Kg, even more preferably comprised from 295 mOsm/Kg to 328 mOsm/Kg, for example 300 mOsm/Kg). The present composition is therefore highly modulable depending on how it will be used.

As far as the measurement of osmolality is concerned, such parameter can be measured experimentally by means of an osmometer. By way of example, an osmometer that can be used for such measurement is a "LOSER" (manufacturer) AUTOMATIC DIGITAL OSMOMETER, model "15".

The present composition is a composition for use in a method for the preventive or curative treatment of a disorder or a disease associated with a corneal epithelial defect and/or a conjunctival defect in a subject. The disorder or disease may be hypotonic, isotonic or hypertonic. By way of example, the disorder or the disease is selected from among the group comprising or, alternatively, consisting of dry keratoconjunctivitis (hypotonic), dry eye syndrome (isotonic), and corneal oedema (hypertonic).

The inventors of the present invention have surprisingly found that, in the absence of a chelating agent (such as for example EDTA, or salts thereof) and in the presence of the two viscosity adjusting agents, the present composition is markedly more tolerable with respect to the known compositions: on the one hand, the absence of chelating agents ensures that the present composition does not subtract ions from the cornea or from the conjunctiva, and therefore it does not abstract and does not burn when applied to the cornea; on the other hand, the presence of the viscosity adjusting agents allows to obtain extremely favourable conditions for the physiology of the eye, especially since it guarantees that the present composition adheres to the cornea, and remains therein in a stable and lasting manner.

By way of example, the chelating agents conventionally used in the ophthalmologic field are citrate salts, citric acid, diethylene diamine tetraacetic acid (EDTA; CAS N° 60-00-4), triethylene glycol diamine tetraacetic acid (EGTA, CAS N° 67-42-5), or combinations thereof. All these chelating agents are not present in the composition and/or mixture of the present invention.

Hyaluronic acid (CAS N° 9004-61-9) is one of the fundamental components of the connective tissues of human beings, and other mammals. The inner portion of the vitreous body of the eye is rich in hyaluronic acid.

Advantageously, in the present composition hyaluronic acid (a.1) is linear and besides performing the viscosity adjusting action (i.e. it increases the viscosity of the composition itself), it also performs a corneal epithelium reconstruction/regeneration function.

The salt of hyaluronic acid or hyaluronate (a.1) could be selected from among the group comprising or, alternatively, consisting of sodium hyaluronate (CAS N° 9067-32-7), potassium hyaluronate (CAS N° 31799-91-4), and mixtures thereof. The sodium hyaluronate that can be used in the present composition is preferably obtained by means of a biotechnological process. Preferably, sodium hyaluronate is in the form of powder or granule, preferably devoid of preservatives.

Hyaluronic acid (linear), hyaluronate, sodium hyaluronate, or mixtures thereof (a.1) has an average molecular weight comprised from 500 kDa to 1,500 kDa, preferably comprised from 600 kDa to 1,200 kDa, more preferably comprised from 700 kDa to 1,000 kDa, even more preferably from 800 kDa to 900 kDa.

According to an embodiment, the mixture comprises only the two viscosity adjusting agents (a.1) and (a.2) mentioned previously, i.e. the first viscosity adjusting agent (a.1) selected from among the group comprising or, alternatively, consisting of hyaluronic acid, a salt thereof and mixtures thereof; and the second viscosity adjusting agent (a.2) comprising or, alternatively, consisting of the ethylene oxide/propylene oxide block copolymer,

The mixture comprises a first viscosity adjusting agent (a.1) selected from among the group comprising or, alternatively, consisting of hyaluronic acid, a salt thereof and mixtures thereof; and only one second viscosity adjusting agent (a.2) comprising or, alternatively, consisting of the ethylene oxide/propylene oxide block copolymer, In other words, there are no two different second viscosity adjusting agents (a.2). The first viscosity adjusting agent (a.1) is hyaluronic acid or a salt thereof and the second viscosity adjusting agent (a.2) is the ethylene oxide/propylene oxide block copolymer, copolymer CAS N° 9003-11-6.

A weight ratio [second viscosity adjusting agent (a.2)]: [first viscosity adjusting agent (a.1)] - for example an ethylene oxide/propylene oxide block copolymer:hyaluronate ratio - is preferably comprised from 15 to 50, preferably from 20 to 45, even more preferably from 23 to 40.

According to a preferred embodiment, a weight ratio [second viscosity adjusting agent (a.2)]:[first viscosity adjusting agent (a.1)] - for example an ethylene oxide/propylene oxide block copolymer:hyaluronate ratio - is comprised from 30 to 40, or it is comprised from 22 to 28.

The second viscosity adjusting agent (a.2) comprises, or alternatively, consists of an ethylene oxide/propylene oxide block copolymer, or a copolymer (ethylene oxide)-block-(propylene oxide)-block-(ethylene oxide).

The ethylene oxide/propylene oxide block copolymer, or (ethylene oxide)-block-(propylene oxide)-block-(ethylene oxide), comprises a percentage by weight comprised from 60% to 90%, preferably comprised from 65% to 85%, even more preferably comprised from 71,5% to 74,9%, of ethylene oxide.

Preferably, the ethylene oxide-propylene oxide block copolymer is the copolymer CAS N° 9003-11-6. By way of example, a block copolymer that can be used in the present invention is known under the trade names Pluronic F-127, Lutrol F 127, Poloxamer 407, or Kolliphor^{®} P 407 (the latter produced by BASF AG).

Such block copolymer is known to have gelling properties, when present in an aqueous solution at a percentage by weight comprised from 10% to 15% and at a temperature comprised from 34°C to 38°C.

Advantageously, in the present composition the two viscosity adjusting agents (a.1) and (a.2) exert a synergistic action given that, at contact with the cornea or with the conjunctiva, at a temperature of about 37°C, they are capable of developing favourable mucoadhesive properties based on a sol/gel transition activated by temperature and reversible (thermo-reversible), and therefore to remain longer after application with respect to the compositions of the prior art.

This synergistic effect is particularly marked when the weight ratio between the two viscosity adjusting agents (a.2) and (a.1) is comprised from 15 to 50, as illustrated in the preceding embodiments.

Mucoadhesion is a property of the present composition which proved to be independent with respect to the viscosity of such composition: as a matter of fact, the inventors of the present invention were able to empirically verify that the composition subject of the present invention proved to be more mucoadhesive even with respect to compositions of the prior art having the same viscosity. Therefore, such preliminary experimental evidence suggests an interaction of the present composition with mucin (the latter being an epithelial tissue excretion product).

Moreover, according to a further advantageous aspect, a combination of viscosity adjusting agents allows to obtain a composition in the form of gel, cream or ointment at room temperature, which is more easily controllable during dispensing and dosing. Nevertheless, when placed at contact with the cornea, this gel composition undergoes a state transition induced by the temperature of the eye which makes it more fluid, and therefore capable of being distributed better on the surface of the eye, and of persisting in such position by virtue of the mucoadhesive action discussed previously.

The buffer agent (b), used in combination with (a) in the mixture of the present invention, is a mixture of boric acid (H₃BO₃) and tris(hydroxymethyl)aminomethane (trometamol or tromethamine; CAS N° 77-86-1), configured to adjust a pH value comprised from 6,5 to 8 ±0,1, preferably comprised from 6,8 to 7,5, even more preferably of about 7,0±0,1.

The mixture of boric acid and tris(hydroxymethyl)aminomethane, is capable of maintaining the pH value of the present composition in a range physiologically compatible with an eye contact, and it was also selected so as to contribute to the osmolality value required by the composition.

In this description, the expression "borate buffer" refers to an equimolar mixture of boric acid and a salt thereof, preferably a sodium salt thereof, even more preferably sodium tetraborate.

A boric acid (CAS N° 10043-35-3) usable in an embodiment of the present invention could be pure and in the form of powder or granule, and have a solubility in water at 20°C of 46,5 g/l. A sodium tetraborate usable in an embodiment of the present invention could be in the form of pure salt.

The pharmaceutical grade additive or excipient (c) of the mixture, when present, comprises or, alternatively, consists of at least one agent for adjusting the tonicity of the composition.

The tonicity adjusting agent could be selected from among the group comprising or, alternatively, consisting of hydrochloric acid, sorbitol (CAS N° 50-70-4), and mixtures thereof.

A hydrochloric acid that can be used in the present composition is preferably an aqueous solution of such acid, for example at a concentration of 1 mol/l.

A sorbitol that can be used in the present composition is preferably a water-soluble white powder.

The tonicity adjusting agent could also be selected from among the group comprising or, alternatively, consisting of sodium chloride, mannitol and mixtures thereof.

According to a preferred embodiment, the composition comprising or, alternatively, consisting of:
(a.1) a first viscosity adjusting agent selected from among the group comprising or, alternatively consisting of hyaluronic acid, hyaluronate (for example sodium hyaluronate and/ potassium hyaluronate), and the mixtures thereof;
(a.2) a second viscosity adjusting agent comprising or, alternatively consisting of the ethylene oxide/propylene oxide block copolymer (for example CAS N° 9003-11-6);
(b) a mixture of boric acid and tris(hydroxymethyl)aminomethane
(c) at least one agent for adjusting the tonicity of the composition selected from among the group comprising or, alternatively consisting of hydrochloric acid, sorbitol, mannitol and mixtures thereof;
(d) distilled water.

Preferably, the composition may comprise or, alternatively, consist of the following components (expressed as a percentage by weight of the component with respect to the total weight of the composition):
(a.1) hyaluronic acid, sodium hyaluronate, potassium hyaluronate or the mixtures thereof at an amount comprised from 0,05% to 1,5%, preferably comprised from 0,1% to 1%, even more preferably comprised from 0,2% to 0,6%, for example 0,4%;
(a.2) the copolymer CAS N° 9003-11-6 at an amount comprised from 5% to 25%, preferably comprised from 7% to 20%, even more preferably comprised from 9% to 16%, for example 10%, 13,5% or 15%;
(b) a mixture of boric acid and tris(hydroxymethyl)aminomethane, preferably at ar amount comprised from 0,01% to 1%, preferably comprised from 0,035% to 0,8%, more preferably comprised from 0,05% to 0,7%, even more preferably comprised from 0,08% to 0,5%, for example comprised from 0,1% to 0,32%; more preferably wherein said boric acid is present at an amount comprised from 0,01% to 0,05% by weight, preferably from 0,02% to 0,04% by weight, even more preferably from 0,03% to 0,035% by weight, with respect to the total weight of said composition, and wherein said tris(hydroxymethyl)aminomethane is present at an amount comprised from 0,01% to 0,12% by weight, preferably from 0,04% to 0,1% by weight, even more preferably from 0,06% to 0,08% by weight, with respect to the total weight of said composition;
(d) a tonicity adjusting agent at an amount comprised from 0,01% to 22%, preferably comprised from 0,03% to 18%, even more preferably comprised from 0,075% to 15%, for example 1N hydrochloric acid at an amount comprised from 0,08% to 1,3% or sorbitol and/or mannitol at a percentage comprised from 12% to 15% by weight;
(e) distilled water up to a total weight of said composition of 100 g.

The composition, subject of the present invention, is characterised in that it causes discomfort - following administration of such composition in an eye of a subject - that lasts lesser (and/or less intense) with respect to a duration of a discomfort following administration of a corresponding composition comprising at least one chelating agent.

As regards the definition of a "corresponding composition comprising at least one chelating agent", reference is made in particular to the compositions exemplified in Example 4, Example 5 or Example 6.

The aforementioned lesser duration of the discomfort is advantageously lesser (in minutes) by a percentage comprised from 10% to 90%, preferably comprised from 20% to 80%, even more preferably comprised from 30% and 70%, with respect to the duration of discomfort following the administration of the corresponding composition comprising the at least one chelating agent.

Forming an object of the present invention is a composition as defined in Table 1 of Example 1, in Table 2 of Example 2, or in Table 3 of Example 3.

Reported hereinafter are some examples of the present invention, provided by way of non-limiting example.

### EXAMPLES.

### Example 1: Composition subject of the present invention - Isotonic gel. (not according to the claims)

A composition was prepared using the components at the percentages (percentages by weight of the component with respect to the total weight of the composition) indicated in Table 1 below.

**Table 1**

| COMPONENT | AMOUNT % |
|---|---|
| Ethylene oxide/propylene oxide block copolymer (Kolliphor^{®} P 407) | 15,0 |
| Boric acid | 0,200 |
| Sodium tetraborate | 0,033 |
| Hydrochloric acid (1N) | 0,090 |
| Sodium hyaluronate | 0,400 |
| Water for injectables | up to 100 g |

Such composition has a pH value of 7,1, and a measured osmolality of 300 mOsm/Kg.

### Example 2: Composition subject of the present invention - Hypotonic gel. (not according to the claims)

A composition was prepared using the components at the percentages (percentages by weight of the component with respect to the total weight of the composition) indicated in Table 2 below.

**Table 2**

| COMPONENT | AMOUNT % |
|---|---|
| Ethylene oxide/propylene oxide block copolymer (Kolliphor^{®} P 407) | 13,5 |
| Boric acid | 0,275 |
| Sodium tetraborate | 0,033 |
| Hydrochloric acid (1N) | 0,110 |
| Sodium hyaluronate | 0,400 |
| Water for injectables | up to 100 g |

Such composition has a pH value of 7,0, and a measured osmolality of 250 mOsm/Kg.

### Example 3: Composition subject of the present invention - Hypertonic gel.

Compositions were prepared using the components at the percentages (percentages by weight of the component with respect to the total weight of the composition) indicated in Table 3, Table 3A and Table 3B below.

**Table 3**

| COMPONENT | AMOUNT % |
|---|---|
| Ethylene oxide/propylene oxide block copolymer (Kolliphor^{®} P 407) | 10,0 |
| Boric acid | 0,033 |
| tris(hydroxymethyl) aminomethane (Trometamol) | 0,076 |
| Sorbitol | 13,50 |
| Sodium hyaluronate | 0,400 |
| Water for injectables | up to 100 g |

Such composition has a pH value of 7,0 ± 0,1, and a measured osmolality of 840 mOsm/Kg.

**Table 3A**

| COMPONENT | AMOUNT % |
|---|---|
| Ethylene oxide/propylene oxide block copolymer (Kolliphor^{®} P 407) | 10,0 |
| Boric acid | 0,033 |
| tris(hydroxymethyl) aminomethane (Trometamol) | 0,076 |
| Mannitol | 15,00 |
| Sodium hyaluronate | 0,400 |
| Water for injectables | up to 100 g |

Such composition has a pH value of 7,1 ± 0,1, and a measured osmolality of 890 mOsm/Kg.

**Table 3B**

| COMPONENT | AMOUNT % |
|---|---|
| Ethylene oxide/propylene oxide block copolymer (Kolliphor^{®} P 407) | 10,0 |
| Boric acid | 0,033 |
| tris(hydroxymethyl) aminomethane (Trometamol) | 0,076 |
| Sorbitol | 13,50 |
| Sodium hyaluronate | 0,200 |
| Water for injectables | up to 100 g |

Such composition has a pH value of 7,0 ± 0,1, and a measured osmolality of 870 mOsm/Kg.

### Example 4: Comparison composition - Isotonic eye drops. (not according to the claims)

A composition was prepared using the components at the percentages (percentages by weight of the component with respect to the total weight of the composition) indicated in Table 4 below.

**Table 4**

| COMPONENT | AMOUNT % |
|---|---|
| Ethylene oxide/propylene oxide block copolymer (Kolliphor^{®} P 407) | 15,0 |
| Boric acid | 0,033 |
| Sodium tetraborate decahydrate | 0,055 |
| EDTA | 0,110 |
| Sodium hyaluronate (PM=0,33 MDa) | 0,400 |
| Water for injectables | up to 100 g |

Such composition has a pH value of 7,0, and a measured osmolality of 293 mOsm/Kg, such values (of pH and of osmolality) being average values measured from two different samples.

### Example 5: Comparison composition - Hypotonic eye drops. (not according to the claims)

A composition was prepared using the components at the percentages (percentages by weight of the component with respect to the total weight of the composition) indicated in Table 5 below.

**Table 5**

| COMPONENT | AMOUNT % |
|---|---|
| Ethylene oxide/propylene oxide block copolymer (Kolliphor^{®} P 407) | 13,5 |
| Boric acid | 0,033 |
| Sodium tetraborate decahydrate | 0,055 |
| EDTA | 0,110 |
| Sodium hyaluronate (PM=0,33 MDa) | 0,400 |
| Water for injectables | up to 100 g |

Such composition has a pH value of 7,0, and a measured osmolality of 238 mOsm/Kg, such values (of pH and of osmolality) being average values measured from two different samples.

### Example 6: Comparison composition - Hypertonic eye drops.

A composition was prepared using the components at the percentages (percentages by weight of the component with respect to the total weight of the composition) indicated in Table 6 below.

**Table 6**

| COMPONENT | AMOUNT % |
|---|---|
| Ethylene oxide/propylene oxide block copolymer (Kolliphor^{®} P 407) | 15,0 |
| Boric acid | 0,033 |
| Tromethamine | 0,120 |
| EDTA | 0,110 |
| Sorbitol | 13,50 |
| Sodium hyaluronate (PM=0,33 MDa) | 0,400 |
| Water for injectables | up to 100 g |

Such composition has a pH value of 7,0, and a measured osmolality of 238 mOsm/Kg, such values (of pH and of osmolality) being average values measured from two different samples.

### Example 7: Comparison between the compositions subject of the present invention and the comparison compositions.

The compositions, subject of the present invention, (prepared according to Example 1, Example 2 and Example 3) were compared with the corresponding compositions comprising at least one chelating agent (prepared according to Example 4, Example 5 and Example 6), comprising **in** particular EDTA as chelating agent.

The duration of the discomfort following the administration of the compositions mentioned above was timed from the moment of instillation of each composition into the eye. The durations reported in Table 7 below are average values calculated on the observation of five subjects.

**Table 7**

| INVENTION | | COMPARISON | | Reduced duration of discomfort |
|---|---|---|---|---|
| | Discomfort duration (min) | | Discomfort duration (min) | |
| Isotonic gel (E.g. 1) | 2 | Isotonic eye drops (E.g. 4) | 3 | 33% |
| Hypotonic gel (E.g. 2) | 2 | Hypotonic eye drops (E.g. 5) | 6 | 67% |
| Hypertonic gel (E.g. 3) | 1 | Hypertonic eye drops (E.g. 6) | 2 | 50% |

As well exemplified by the table above, the compositions subject of the present invention allow to reduce - in a significant way - the discomfort time of the subject with respect to the compositions comprising the chelating agent (EDTA).

The higher percentage reduction (67%) can be found in the hypotonic compositions, and this is consistent with the identical percentages of EDTA in the compositions of Example 4, Example 5 and Example 6.

More precisely, in a condition of deficient tonicity of the composition with respect to the tear liquid, the astringency and discomfort perceived by the subject, and generated by the presence of the chelating agent, will be higher where the amount of available ions that can be complexed by such agent is smaller. Thus, after all the monovalent ions available in the comparison composition (mainly sodium ions) have been complexed, EDTA complexes the bivalent ions of the tear fluid, thus rapidly generating the hypotonic areas discussed previously which will last for the entire duration of the discomfort.

### Example 8: Comparison between the viscosity of the isotonic Gel (Example 1) subject of the present invention at different temperatures in an accelerated stability test.

The viscosity behaviour of the composition of Example 1 (isotonic gel) was tested in an accelerated stability test at +40°C and 75% RH (relative humidity) at different temperatures.

The results obtained are shown in Table 8 below. The viscosity values were measured with a static viscometer determination system of the BROOKFIELD type, bearing a spindle N° 7.

**Table 8**

| Viscosity | Isotonic gel (E.g. 1) | | |
|---|---|---|---|
| mPa.S | t0 | 3 months | 6 months |
| + 20°C | 166 | 175 | 176 |
| +37°C | 18798 | 19224 | 18560 |

From the previous Table 8 it can be observed that - at a temperature of 20°C - the viscosity of the isotonic composition subject of the invention tends to increase over time, although in a less marked way from the third to the sixth month from the start of the test. Without wishing to provide any scientific explanation of the phenomenon, this increase in viscosity could be due to a slight initial evaporation of the carrier.

However, one of the innovative aspects of the composition subject of the present invention lies in the increase (greater than 100 times) in viscosity from 20°C to 37°C. Such experimental data confirms that the present composition is a liquid flowing at room temperature (20°C) but, when administered and brought to a physiological temperature of the eye (37°C), it becomes extremely viscous and poorly flowable. Thus, besides a more marked barrier effect for the insulation of corneal damage, the batting of the eyelids and tear washing have more difficulty in causing an expulsion of the present composition with respect to other compositions of the prior art for which there is no variation in viscosity as a function of the temperature so marked.

It should be further observed that stability tests corresponding to the one discussed above in this example were conducted for the compositions comprising EDTA (Example 4, Example 5 and Example 6). Also, in such tests, the trend at 3 months and 6 months follows the trends observed in Table 8.

Thus, it can be deduced that, although the absence of EDTA in the compositions subject of the present invention is a negative factor as regards *in vivo* gelation, the compositions subject of the present invention have at least equal performance also in terms of viscosity as a function of temperature with respect to the corresponding compositions in which EDTA is present for comparable periods of time.

E(n) preferred embodiments of the present invention are illustrated below:
E1. An ophthalmic composition, devoid of chelating agents, comprising a mixture comprising, or alternatively, consisting of:
   (a) at least two viscosity adjusting agents; a first viscosity adjusting agent (a.1) selected from among the group comprising or, alternatively, consisting of hyaluronic acid, a salt thereof and mixtures thereof; a second viscosity adjusting agent (a.2) comprising or, alternatively, consisting of an ethylene oxide/propylene oxide block copolymer,
   (b) a buffer agent which is a mixture of boric acid and tris(hydroxymethyl)aminomethane for adjusting the pH of the composition to a pH value comprised from 6,5 to 8 ± 0,1,
   (c) preferably one or more pharmaceutical grade additives and excipients, and
   (d) water;
   said composition being for use in a method for the preventive or curative treatment of a disorder or a disease associated with a corneal epithelial defect and/or a conjunctival defect in a subject.
E2. The composition according to E1, wherein: said boric acid is present at an amount comprised from 0,01% to 0,05% by weight, preferably from 0,02% to 0,04% by weight, even more preferably from 0,03% to 0,035% by weight, with respect to the total weight of said composition, and wherein said tris(hydroxymethyl)aminomethane is present at an amount comprised from 0,01% to 0,12% by weight, preferably from 0,04% to 0,1% by weight, even more preferably from 0,06% to 0,08% by weight, with respect to the total weight of said composition.
E3. The composition according to any one of E1-E2, wherein said pH value is comprised from 6,8 to 7,5, said composition has an osmolality comprised from 200 mOsm/Kg to 950 mOsm/Kg, wherein hyaluronic acid, sodium hyaluronate, or mixtures thereof (a.1) has an average molecular weight comprised from 500 kDa to 1,500 kDa, and wherein hyaluronic acid, sodium hyaluronate, or mixtures thereof (a.1) is present at an amount comprised from 0,05% to 1,5% by weight with respect to the total weight of the composition.
E4. The composition for use according to E3, wherein said pH value is of about 7,0±0,1, said composition has an osmolality comprised from 220 mOsm/Kg to 880 mOsm/Kg, preferably comprised from 250 mOsm/Kg to 800 mOsm/Kg, wherein hyaluronic acid, sodium hyaluronate, or mixtures thereof (a.1) has an average molecular weight comprised from 600 kDa to 1,200 kDa, preferably comprised from 700 kDa to 1,000 kDa, even more preferably from 800 kDa to 900 kDa, and wherein hyaluronic acid, sodium hyaluronate, or mixtures thereof (a.1) is present at an amount comprised from 0,1% to 1% by weight, preferably comprised from 0,2% to 0,6% by weight, even more preferably 0,4% by weight, with respect to the total weight of the composition.
E5. The composition for use according to E3 or E4, wherein said composition is:
   - hypotonic, with an osmolality value of about from 225 mOsm/Kg to 275 mOsm/Kg or comprised from 220 mOsm/Kg to 265 mOsm/Kg, preferably comprised from 230 mOsm/Kg to 260 mOsm/Kg, even more preferably comprised from 235 mOsm/Kg to 255 mOsm/Kg, for example 250 mOsm/Kg; or
   - hypertonic, with an osmolality value greater than from 350 mOsm/Kg to 850 mOsm/Kg or comprised from 700 mOsm/Kg to 1,000 mOsm/Kg, preferably comprised from 750 mOsm/Kg to 930 mOsm/Kg, even more preferably comprised from 800 mOsm/Kg to 910 mOsm/Kg, for example 880 mOsm/Kg; or
   - isotonic, with an osmolality value of about from 275 mOsm/Kg to 325 mOsm/Kg or comprised from 270 mOsm/Kg to 335 mOsm/Kg, preferably comprised from 280 mOsm/Kg to 330 mOsm/Kg, even more preferably comprised from 295 mOsm/Kg to 328 mOsm/Kg, for example 300 mOsm/Kg.
E6. The composition for use according to any one of E1-E5, wherein said composition has a by weight ratio [second viscosity adjusting agent (a.2)]:[first viscosity adjusting agent (a.1)] comprised from 15 to 50, preferably from 20 to 45, even more preferably from 23 to 40.
E7. The composition for use according to any one of E1-E6, wherein the ethylene oxide/propylene oxide block copolymer is the CAS N° 9003-11-6 copolymer.
E8. The composition for use according to any one of E1-E7, wherein said composition is devoid of phosphate ions, monohydrogen phosphate ions and/or dihydrogen phosphate ions.
E9. The composition for use according to any one of E1-E8, wherein said mixture comprises or, alternatively consists of:
   (a.1) a first viscosity adjusting agent selected from among the group comprising or, alternatively consisting of hyaluronic acid, a salt thereof and the mixtures thereof;
   (a.2) a second viscosity adjusting agent comprising or, alternatively consisting of the ethylene oxide/propylene oxide block copolymer;
   (b) a mixture of boric acid and tris(hydroxymethyl)aminomethane;
   (c) at least one agent for adjusting the tonicity of said composition selected from among the group comprising or, alternatively consisting of hydrochloric acid, sorbitol, mannitol and mixtures thereof;
   (d) distilled water;
   said composition preferably being devoid of phosphates.
E10. The composition for use according to E9, wherein said composition comprises or, alternatively consists of:
   (a.1) a hyaluronic acid, sodium hyaluronate, or the mixtures thereof at an amount comprised from 0,05% to 1,5%, preferably comprised from 0,1% to 1%, even more preferably comprised from 0,2% to 0,6%, for example 0,4% by weight, with respect to the total weight of the composition;
   (a.2) a CAS N° 9003-11-6 copolymer at an amount comprised from 5% to 25%, preferably comprised from 7% to 20%, even more preferably comprised from 9% to 16%, for example 10%, 13,5% or 15% by weight, with respect to the total weight of the composition;
   (b) a mixture of boric acid and tris(hydroxymethyl)aminomethane, wherein said boric acid is present at an amount comprised from 0,01% to 0,05% by weight, preferably from 0,02% to 0,04% by weight, even more preferably from 0,03% to 0,035% by weight, with respect to the total weight of said composition, and wherein said tris(hydroxymethyl)aminomethane is present at an amount comprised from 0,01% to 0,12% by weight, preferably from 0,04% to 0,1% by weight, even more preferably from 0,06% to 0,08% by weight, with respect to the total weight of said composition;
   (c) a tonicity adjusting agent at an amount comprised from 0,01% to 22%, preferably comprised from 0,03% to 18%, even more preferably comprised from 0,075% to 15%, for example 1N hydrochloric acid at an amount comprised from 0,08% to 1,3% or sorbitol and/or mannitol at an amount comprised from 12% to 15% by weight, with respect to the total weight of the composition;
   (d) distilled water up to a total weight of said composition of 100 g.
E11. The composition for use according to any one of E1-E10, wherein said composition is liquid eye drops, or gel, cream or ointment eye drops.

## Claims

1. An ophthalmic composition, devoid of chelating agents, comprising a mixture comprising, or alternatively, consisting of:
(a) at least two viscosity adjusting agents; a first viscosity adjusting agent (a.1) selected from among the group comprising or, alternatively, consisting of hyaluronic acid, a salt thereof and mixtures thereof; a second viscosity adjusting agent (a.2) comprising or, alternatively, consisting of an ethylene oxide/propylene oxide block copolymer,
(b) a buffer agent which is a mixture of boric acid and tris(hydroxymethyl)aminomethane for adjusting the pH of the composition to a pH value comprised from 6,5 to 8 ± 0,1,
(c) preferably one or more pharmaceutical grade additives and excipients, and
(d) water;
said composition being for use in a method for the preventive or curative treatment of a disorder or a disease associated with a corneal epithelial defect and/or a conjunctival defect in a subject.

2. The composition for use according to claim 1, wherein: said boric acid is present at an amount comprised from 0,01% to 0,05% by weight, preferably from 0,02% to 0,04% by weight, even more preferably from 0,03% to 0,035% by weight, with respect to the total weight of said composition, and wherein said tris(hydroxymethyl)aminomethane is present at an amount comprised from 0,01% to 0,12% by weight, preferably from 0,04% to 0,1% by weight, even more preferably from 0,06% to 0,08% by weight, with respect to the total weight of said composition.

3. The composition for use according to any one of the preceding claims, wherein said pH value is comprised from 6,8 to 7,5, said composition has an osmolality comprised from 200 mOsm/Kg to 950 mOsm/Kg, wherein hyaluronic acid, sodium hyaluronate, or mixtures thereof (a.1) has an average molecular weight comprised from 500 kDa to 1,500 kDa, and wherein hyaluronic acid, sodium hyaluronate, or mixtures thereof (a.1) is present at an amount comprised from 0,05% to 1,5% by weight with respect to the total weight of the composition.

4. The composition for use according to claim 3, wherein said pH value is of about 7,0±0,1, said composition has an osmolality comprised from 220 mOsm/Kg to 880 mOsm/Kg, preferably comprised from 250 mOsm/Kg to 800 mOsm/Kg, wherein hyaluronic acid, sodium hyaluronate, or mixtures thereof (a.1) has an average molecular weight comprised from 600 kDa to 1,200 kDa, preferably comprised from 700 kDa to 1,000 kDa, even more preferably from 800 kDa to 900 kDa, and wherein hyaluronic acid, sodium hyaluronate, or mixtures thereof (a.1) is present at an amount comprised from 0,1% to 1% by weight, preferably comprised from 0,2% to 0,6% by weight, even more preferably 0,4% by weight, with respect to the total weight of the composition.

5. The composition for use according to claim 3 or 4, wherein said composition is:
- hypotonic, with an osmolality value of about from 225 mOsm/Kg to 275 mOsm/Kg or comprised from 220 mOsm/Kg to 265 mOsm/Kg, preferably comprised from 230 mOsm/Kg to 260 mOsm/Kg, even more preferably comprised from 235 mOsm/Kg to 255 mOsm/Kg, for example 250 mOsm/Kg; or
- hypertonic, with an osmolality value greater than from 350 mOsm/Kg to 850 mOsm/Kg or comprised from 700 mOsm/Kg to 1,000 mOsm/Kg, preferably comprised from 750 mOsm/Kg to 930 mOsm/Kg, even more preferably comprised from 800 mOsm/Kg to 910 mOsm/Kg, for example 880 mOsm/Kg; or
- isotonic, with an osmolality value of about from 275 mOsm/Kg to 325 mOsm/Kg or comprised from 270 mOsm/Kg to 335 mOsm/Kg, preferably comprised from 280 mOsm/Kg to 330 mOsm/Kg, even more preferably comprised from 295 mOsm/Kg to 328 mOsm/Kg, for example 300 mOsm/Kg.

6. The composition for use according to any one of the preceding claims, wherein said composition has a by weight ratio [second viscosity adjusting agent (a.2)]:[first viscosity adjusting agent (a.1)] comprised from 15 to 50, preferably from 20 to 45, even more preferably from 23 to 40.

7. The composition for use according to any one of the preceding claims, wherein the ethylene oxide/propylene oxide block copolymer is the CAS N° 9003-11-6 copolymer.

8. The composition for use according to any one of the preceding claims, wherein said composition is devoid of phosphate ions, monohydrogen phosphate ions and/or dihydrogen phosphate ions.

9. The composition for use according to any one of the preceding claims, wherein said mixture comprises or, alternatively consists of:
(a.1) a first viscosity adjusting agent selected from among the group comprising or, alternatively consisting of hyaluronic acid, a salt thereof and the mixtures thereof;
(a.2) a second viscosity adjusting agent comprising or, alternatively consisting of the ethylene oxide/propylene oxide block copolymer;
(b) a mixture of boric acid and tris(hydroxymethyl)aminomethane;
(c) at least one agent for adjusting the tonicity of said composition selected from among the group comprising or, alternatively consisting of hydrochloric acid, sorbitol, mannitol and mixtures thereof;
(d) distilled water;
said composition preferably being devoid of phosphates.

10. The composition for use according to the preceding claim wherein said composition comprises or, alternatively consists of:
(a.1) a hyaluronic acid, sodium hyaluronate, or the mixtures thereof at an amount comprised from 0,05% to 1,5%, preferably comprised from 0,1% to 1%, even more preferably comprised from 0,2% to 0,6%, for example 0,4% by weight, with respect to the total weight of the composition;
(a.2) a CAS N° 9003-11-6 copolymer at an amount comprised from 5% to 25%, preferably comprised from 7% to 20%, even more preferably comprised from 9% to 16%, for example 10%, 13,5% or 15% by weight, with respect to the total weight of the composition;
(b) a mixture of boric acid and tris(hydroxymethyl)aminomethane, wherein said boric acid is present at an amount comprised from 0,01% to 0,05% by weight, preferably from 0,02% to 0,04% by weight, even more preferably from 0,03% to 0,035% by weight, with respect to the total weight of said composition, and wherein said tris(hydroxymethyl)aminomethane is present at an amount comprised from 0,01% to 0,12% by weight, preferably from 0,04% to 0,1% by weight, even more preferably from 0,06% to 0,08% by weight, with respect to the total weight of said composition;
(c) a tonicity adjusting agent at an amount comprised from 0,01% to 22%, preferably comprised from 0,03% to 18%, even more preferably comprised from 0,075% to 15%, for example 1N hydrochloric acid at an amount comprised from 0,08% to 1,3% or sorbitol and/or mannitol at an amount comprised from 12% to 15% by weight, with respect to the total weight of the composition;
(d) distilled water up to a total weight of said composition of 100 g.

11. The composition for use according to any one of the preceding claims, wherein said composition is liquid eye drops, or gel, cream or ointment eye drops.

## Patentansprüche

1. Ophthalmische Zusammensetzung ohne Chelatoren, umfassend eine Mischung, umfassend oder alternativ bestehend aus:
(a) mindestens zwei Viskositätsregulierungsmittel(n); ein(em) erstes(n) Viskositätsregulierungsmittel (a.1) ausgewählt aus der Gruppe umfassend oder alternativ bestehend aus Hyaluronsäure, ein(em) Salz davon und Mischungen davon; ein(em) zweites(en) Viskositätsregulierungsmittel (a.2) umfassend oder alternativ bestehend aus ein(em) Ethylenoxid/Propylenoxid-Blockcopolymer,
(b) ein(em) Puffermittel, das eine Mischung aus Borsäure und Tris(hydroxymethyl)aminomethan ist, um den pH-Wert der Zusammensetzung auf einen pH-Wert im Bereich von 6,5 bis 8 ± 0,1 zu regulieren,
(c) vorzugsweise einen(em) oder mehrere(n) pharmazeutische(n) Zusatzstoffe(n) und Hilfsstoffe(n) und
(d) Wasser;
wobei die Zusammensetzung zur Verwendung in einem Verfahren zur präventiven oder kurativen Behandlung einer Störung oder einer Erkrankung vorgesehen ist, die mit einem Hornhautepitheldefekt und/oder einem Bindehautdefekt bei einem Patienten verbunden ist.

2. Zusammensetzung zur Verwendung nach Anspruch 1, wobei: die Borsäure in einer Menge im Bereich von 0,01 bis 0,05 Gew.- %, vorzugsweise von 0,02 bis 0,04 Gew.- %, noch bevorzugter von 0,03 bis 0,035 Gew.- %, bezogen auf das Gesamtgewicht der Zusammensetzung, vorhanden ist und wobei das Tris(hydroxymethyl)aminomethan in einer Menge im Bereich von 0,01 bis 0,12 Gew.- %, vorzugsweise von 0,04 bis 0,1 Gew.- %, noch bevorzugter von 0,06 bis 0,08 Gew.- %, bezogen auf das Gesamtgewicht der Zusammensetzung, vorhanden ist.

3. Zusammensetzung zur Verwendung nach einem der vorhergehenden Ansprüche, wobei der pH-Wert im Bereich von 6,8 bis 7,5 umfasst ist, die Zusammensetzung eine Osmolalität im Bereich von 200 mOsm/kg bis 950 mOsm/kg aufweist, wobei Hyaluronsäure, Natriumhyaluronat oder Mischungen davon (a.1) ein durchschnittliches Molekulargewicht im Bereich von 500 kDa bis 1.500 kDa aufweist und wobei Hyaluronsäure, Natriumhyaluronat oder Mischungen davon (a.1) in einer Menge im Bereich von 0,05 bis 1,5 Gew.- %, bezogen auf das Gesamtgewicht der Zusammensetzung, vorhanden ist.

4. Zusammensetzung zur Verwendung nach Anspruch 3, wobei der pH-Wert etwa 7,0±0,1 beträgt, die Zusammensetzung eine Osmolalität im Bereich von 220 mOsm/kg bis 880 mOsm/kg, vorzugsweise im Bereich von 250 mOsm/kg bis 800 mOsm/kg aufweist, wobei Hyaluronsäure, Natriumhyaluronat oder Mischungen davon (a.1) ein durchschnittliches Molekulargewicht im Bereich von 600 kDa bis 1.200 kDa, vorzugsweise im Bereich von 700 kDa bis 1.000 kDa, noch bevorzugter von 800 kDa bis 900 kDa aufweist, und wobei Hyaluronsäure, Natriumhyaluronat oder Mischungen davon (a.1) in einer Menge im Bereich von 0,1 bis 1 Gew.- %, vorzugsweise im Bereich von 0,2 bis 0,6 Gew.- %, noch bevorzugter 0,4 Gew.- %, bezogen auf das Gesamtgewicht der Zusammensetzung, vorhanden ist.

5. Zusammensetzung zur Verwendung nach Anspruch 3 oder 4, wobei die Zusammensetzung ist:
- hypotonisch, mit einem Osmolalitätswert von etwa 225 mOsm/kg bis 275 mOsm/kg oder im Bereich von 220 mOsm/kg bis 265 mOsm/kg, vorzugsweise im Bereich von 230 mOsm/kg bis 260 mOsm/kg, noch bevorzugter im Bereich von 235 mOsm/kg bis 255 mOsm/kg, beispielweise 250 mOsm/kg; oder
- hypertonisch, mit einem Osmolalitätswert größer als 350 mOsm/kg bis 850 mOsm/kg oder im Bereich von 700 mOsm/kg bis 1000 mOsm/kg, vorzugsweise im Bereich von 750 mOsm/kg bis 930 mOsm/kg, noch bevorzugter im Bereich von 800 mOsm/kg bis 910 mOsm/kg, beispielweise 880 mOsm/kg; oder
- isotonisch, mit einem Osmolalitätswert von etwa 275 mOsm/kg bis 325 mOsm/kg oder im Bereich von 270 mOsm/kg bis 335 mOsm/kg, vorzugsweise im Bereich von 280 mOsm/kg bis 330 mOsm/kg, noch bevorzugter im Bereich von 295 mOsm/kg bis 328 mOsm/kg, beispielweise 300 mOsm/kg.

6. Zusammensetzung zur Verwendung nach einem der vorhergehenden Ansprüche, wobei die Zusammensetzung ein Gewichtsverhältnis [zweites Viskositätsregulierungsmittel (a.2)]:[erstes Viskositätsregulierungsmittel (a.1)] im Bereich von 15 bis 50, vorzugsweise von 20 bis 45, noch bevorzugter von 23 bis 40 aufweist.

7. Zusammensetzung zur Verwendung nach einem der vorhergehenden Ansprüche, wobei das Ethylenoxid/Propylenoxid-Blockcopolymer das CAS Nr. 9003-11-6-Copolymer ist.

8. Zusammensetzung zur Verwendung nach einem der vorhergehenden Ansprüche, wobei die Zusammensetzung ohne Phosphationen, Monohydrogenphosphationen und/oder Dihydrogenphosphationen ist.

9. Zusammensetzung zur Verwendung nach einem der vorhergehenden Ansprüche, wobei die Mischung umfasst oder alternativ besteht aus:
(a.1) ein(em) erstes(n) Viskositätsregulierungsmittel, ausgewählt aus der Gruppe, umfassend oder alternativ bestehend aus Hyaluronsäure, ein(em) Salz davon und die(den) Mischungen davon;
(a.2) ein(em) zweites(n) Viskositätsregulierungsmittel, das das Ethylenoxid/Propylenoxid-Blockcopolymer umfasst oder alternativ daraus besteht;
(b) eine(r) Mischung aus Borsäure und Tris(hydroxymethyl)aminomethan;
(c) mindestens ein(em) Mittel zur Regulierung der Tonizität der Zusammensetzung, ausgewählt aus der Gruppe umfassend oder alternativ bestehend aus Salzsäure, Sorbit, Mannit und Mischungen davon;
(d) destilliertes(m) Wasser;
wobei die Zusammensetzung vorzugsweise ohne Phosphate ist.

10. Zusammensetzung zur Verwendung nach dem vorhergehenden Anspruch, wobei die Zusammensetzung umfasst oder alternativ besteht aus:
(a.1) eine(r) Hyaluronsäure, Natriumhyaluronat oder Mischungen davon in einer Menge im Bereich von 0,05 % bis 1,5 %, vorzugsweise im Bereich von 0,1 % bis 1 %, noch bevorzugter im Bereich von 0,2 % bis 0,6 %, beispielsweise 0,4 Gew.-%, bezogen auf das Gesamtgewicht der Zusammensetzung;
(a.2) ein(em) CAS Nr. 9003-11-6-Copolymer in einer Menge im Bereich von 5 bis 25 Gew.- %, vorzugsweise im Bereich von 7 bis 20 Gew.- %, noch bevorzugter im Bereich von 9 bis 16 Gew.- %, beispielsweise 10, 13,5 Gew.- % oder 15 Gew.-%, bezogen auf das Gesamtgewicht der Zusammensetzung;
(b) eine(r) Mischung aus Borsäure und Tris(hydroxymethyl)aminomethan, wobei die Borsäure in einer Menge im Bereich von 0,01 bis 0,05 Gew.- %, vorzugsweise von 0,02 bis 0,04 Gew.- %, noch bevorzugter von 0,03 bis 0,035 Gew.- %, bezogen auf das Gesamtgewicht der Zusammensetzung, vorhanden ist und wobei das Tris(hydroxymethyl)aminomethan in einer Menge im Bereich von 0,01 bis 0,12 Gew.- %, vorzugsweise von 0,04 bis 0,1 Gew.- %, noch bevorzugter von 0,06 bis 0,08 Gew.- %, bezogen auf das Gesamtgewicht der Zusammensetzung, vorhanden ist;
(c) ein(em) Tonizitätsregulierungsmittel in einer Menge im Bereich von 0,01 % bis 22 %, vorzugsweise im Bereich von 0,03 % bis 18 %, noch bevorzugter im Bereich von 0,075 % bis 15 %, beispielsweise 1 N Salzsäure in einer Menge im Bereich von 0,08 % bis 1,3 % oder Sorbit und/oder Mannit in einer Menge im Bereich von 12 % bis 15 %, bezogen auf das Gesamtgewicht der Zusammensetzung;
(d) destilliertes(m) Wasser bis zu einem Gesamtgewicht der Zusammensetzung von 100 g.

11. Zusammensetzung zur Verwendung nach einem der vorhergehenden Ansprüche, wobei die Zusammensetzung flüssige Augentropfen oder Gel-, Creme- oder Salben-Augentropfen ist.

## Revendications

1. Composition ophtalmique, dépourvue d'agents chélateurs, comprenant un mélange comprenant, ou alternativement, constitué de :
(a) au moins deux agents d'ajustement de viscosité ; un premier agent d'ajustement de viscosité (a.1) choisi dans le groupe comprenant ou, alternativement, constitué d'acide hyaluronique, un de ses sels et ses mélanges ; un second agent d'ajustement de viscosité (a.2) comprenant ou, alternativement, constitué d'un copolymère à blocs d'oxyde d'éthylène/d'oxyde de propylène,
(b) un agent tampon qui est un mélange d'acide borique et de tris(hydroxyméthyl)aminométhane pour ajuster le pH de la composition à une valeur de pH comprise entre 6,5 et 8 ± 0,1,
(c) de préférence, un ou plusieurs additifs et excipients de qualité pharmaceutique, et
(d) eau ;
ladite composition étant destinée à être utilisée dans une méthode de traitement préventif ou curatif d'un trouble ou d'une maladie associé à un défaut de l'épithélium cornéen et/ou à un défaut conjonctival chez un sujet.

2. Composition destinée à être utilisée selon la revendication 1, dans laquelle : ledit acide borique est présent dans une quantité comprise entre 0,01 et 0,05 % en poids, de préférence entre 0,02 et 0,04 % en poids, plus préférablement encore entre 0,03 et 0,035 % en poids, par rapport au poids total de ladite composition, et dans laquelle ledit tris(hydroxyméthyl)aminométhane est présent dans une quantité comprise entre 0,01 et 0,12 % en poids, de préférence entre 0,04 et 0,1 % en poids, plus préférablement encore entre 0,06 et 0,08 % en poids, par rapport au poids total de ladite composition.

3. Composition destinée à être utilisée selon l'une quelconque des revendications précédentes, dans laquelle ladite valeur de pH est comprise entre 6,8 et 7,5, ladite composition a une osmolalité comprise entre 200 et 950 mOsm/Kg, dans laquelle l'acide hyaluronique, le hyaluronate de sodium, ou leurs mélanges (a.1) ont un poids moléculaire moyen compris entre 500 et 1 500 kDa, et dans laquelle l'acide hyaluronique, le hyaluronate de sodium ou leurs mélanges (a.1) sont présents dans une quantité comprise entre 0,05 et 1,5 % en poids par rapport au poids total de la composition.

4. Composition destinée à être utilisée selon la revendication 3, dans laquelle ladite valeur de pH est d'environ 7,0 ± 0,1, ladite composition a une osmolalité comprise entre 220 et 880 mOsm/Kg, de préférence comprise entre 250 et 800 mOsm/Kg, dans laquelle l'acide hyaluronique, le hyaluronate de sodium, ou leurs mélanges (a.1) ont un poids moléculaire moyen compris entre 600 et 1 200 kDa, de préférence entre 700 et 1 000 kDa, plus préférablement encore entre 800 et 900 kDa, et dans laquelle l'acide hyaluronique, le hyaluronate de sodium ou leurs mélanges (a.1) sont présents dans une quantité comprise entre 0,1 et 1 % en poids, de préférence entre 0,2 et 0,6 % en poids, plus préférablement encore de 0,4 % en poids, par rapport au poids total de la composition.

5. Composition destinée à être utilisée selon la revendication 3 ou 4, dans laquelle ladite composition est :
- hypotonique, avec une valeur d'osmolalité d'environ 225 à 275 mOsm/Kg ou comprise entre 220 et 265 mOsm/Kg, de préférence comprise entre 230 et 260 mOsm/Kg, plus préférablement encore comprise entre 235 et 255 mOsm/Kg, par exemple 250 mOsm/Kg ; ou
- hypertonique, avec une valeur d'osmolalité supérieure à 350 à 850 mOsm/Kg ou comprise entre 700 et 1 000 mOsm/Kg, de préférence comprise entre 750 et 930 mOsm/Kg, plus préférablement encore comprise entre 800 et 910 mOsm/Kg, par exemple 880 mOsm/Kg ; ou
- isotonique, avec une valeur d'osmolalité d'environ 275 à 325 mOsm/Kg ou comprise entre 270 et 335 mOsm/Kg, de préférence comprise entre 280 et 330 mOsm/Kg, plus préférablement encore comprise entre 295 et 328 mOsm/Kg, par exemple 300 mOsm/Kg.

6. Composition destinée à être utilisée selon l'une quelconque des revendications précédentes, dans laquelle ladite composition présente un rapport pondéral [second agent d'ajustement de viscosité (a.2)] : [premier agent d'ajustement de viscosité (a.1 )] compris entre 15 et 50, de préférence entre 20 et 45, plus préférablement encore entre 23 et 40.

7. Composition destinée à être utilisée selon l'une quelconque des revendications précédentes, dans laquelle le copolymère à blocs d'oxyde d'éthylène/d'oxyde de propylène est le copolymère CAS N° 9003-11-6.

8. Composition destinée à être utilisée selon l'une quelconque des revendications précédentes, dans laquelle ladite composition est dépourvue d'ions phosphate, d'ions monohydrogénophosphate et/ou d'ions dihydrogénophosphate.

9. Composition destinée à être utilisée selon l'une quelconque des revendications précédentes, dans laquelle ledit mélange comprend ou, alternativement, est constitué de :
(a.1) un premier agent d'ajustement de viscosité choisi dans le groupe comprenant ou, alternativement, constitué de l'acide hyaluronique, un de ses sels et leurs mélanges ;
(a.2) un second agent d'ajustement de viscosité comprenant ou, alternativement, constitué du copolymère à blocs d'oxyde d'éthylène/d'oxyde de propylène ;
(b) un mélange d'acide borique et de tris(hydroxyméthyl)aminométhane ;
(c) au moins un agent d'ajustement de tonicité de ladite composition choisi dans le groupe comprenant ou, alternativement, constitué de l'acide chlorhydrique, du sorbitol, du mannitol et leurs mélanges ;
(d) eau distillée ;
ladite composition étant de préférence dépourvue de phosphates.

10. Composition destinée à être utilisée selon la revendication précédente, dans laquelle ladite composition comprend ou, alternativement, est constituée de :
(a.1) un acide hyaluronique, un hyaluronate de sodium, ou leurs mélanges dans une quantité comprise entre 0,05 et 1,5 %, de préférence comprise entre 0,1 et 1 %, plus préférablement encore comprise entre 0,2 et 0,6 %, par exemple de 0,4 % en poids, par rapport au poids total de la composition ;
(a.2) un copolymère CAS N° 9003-11-6 dans une quantité comprise entre 5 et 25 %, de préférence entre 7 et 20 %, plus préférablement encore entre 9 et 16 %, par exemple de 10 %, 13,5 % ou 15 % en poids, par rapport au poids total de la composition ;
(b) un mélange d'acide borique et de tris(hydroxyméthyl)aminométhane, dans laquelle ledit acide borique est présent dans une quantité comprise entre 0,01 et 0,05 % en poids, de préférence entre 0,02 et 0,04 % en poids, plus préférablement encore entre 0,03 et 0,035 % en poids, par rapport au poids total de ladite composition, et dans laquelle ledit tris(hydroxyméthyl)aminométhane est présent dans une quantité comprise entre 0,01 et 0,12 % en poids, de préférence entre 0,04 et 0,1 % en poids, plus préférablement encore entre 0,06 et 0,08 % en poids, par rapport au poids total de ladite composition ;
(c) un agent d'ajustement de tonicité dans une quantité comprise entre 0,01 et 22 %, de préférence entre 0,03 et 18 %, plus préférablement encore entre 0,075 et 15 %, par exemple de l'acide chlorhydrique 1 N dans une quantité comprise entre 0,08 et 1,3 % ou du sorbitol et/ou du mannitol dans une quantité comprise entre 12 et 15 % en poids, par rapport au poids total de la composition ;
(d) eau distillée jusqu'à un poids total de ladite composition de 100 g.

11. Composition destinée à être utilisée selon l'une quelconque des revendications précédentes, dans laquelle ladite composition est un collyre liquide ou un collyre sous forme de gel, de crème ou de pommade.
